Europäisches Patentamt

European Patent Office

Office européen des brevets

(11) Publication number: **0 272 669**
**A2**

## EUROPEAN PATENT APPLICATION

(21) Application number: **87118942.9**

(22) Date of filing: **21.12.87**

(51) Int. Cl.⁴: **C12N 1/20** , **C12N 1/38**

(30) Priority: **22.12.86 US 944120**

(43) Date of publication of application:
**29.06.88 Bulletin 88/26**

(84) Designated Contracting States:
**AT CH DE FR GB IT LI SE**

(71) Applicant: **EASTMAN KODAK COMPANY**
**Patent Department 343 State Street**
**Rochester New York 14650(US)**

(72) Inventor: **Lynn, Shirley Yau c/o EASTMAN**
**KODAK COMPANY**
**Patent.Dep.343 State Street**
**Rochester New York 14650(US)**
Inventor: **Noto, Geralyn DeFrank EASTMAN**
**KODAK COMPANY**
**Patent.Dep.343 State Street**
**Rochester New York 14650(US)**

(74) Representative: **Brandes, Jürgen, Dr.rer.nat. et**
**al**
**Thierschstrasse 8**
**D-8000 München 22(DE)**

(54) Fermentation of microorganisms having ice nucleating activity using a defined medium.

(57) A method for the fermentation of microorganisms having a high level of ice nucleating activity is disclosed. The medium used for the fermentation comprises a sugar as the carbon source and α-ketoglutarate or an α-ketoglutarate yielding amino acid as the nitrogen source. In preferred embodiments, the medium also contains phosphate and trace amounts of iron and zinc.

EP 0 272 669 A2

## FERMENTATION OF MICROORGANISMS HAVING ICE NUCLEATING ACTIVITY USING A DEFINED MEDIUM

The present invention relates to a method for the fermentation of microorganisms that have ice nucleating activity.

In U.S. Patent 4,200,228 there is disclosed a method for the making of snow whereby microorganisms are included in droplets that are sprayed into the air. The microorganisms that are used are of the type which are known to promote ice nucleation. As a result, snow can be made at temperatures that are much higher than are ordinarily possible. A typical microorganism that is useful in this process is a Pseudomonad and particularly Pseudomonas syringae.

It is apparent that if this process is to be used on any scale, large amounts of microorganisms are needed. Further, it is desirable that the microorganism be obtained in a dry form so as to facilitate the storage, handling and transport of the material.

The growth conditions for microorganisms that have ice nucleating activity are known in the art. For example, in Maki and Willoughby, Bacteria as Biogenic Sources of Freezing Nuclei, J. Applied Meteorology 17 1049-1053 it is disclosed that the microorganisms such as Pseudomonas syringae are grown in Koser citrate broth at a temperature below 20°C, i. e. 5°C.

In another reference, the microorganisms are grown on a tryptone-yeast extract-glycerol medium which would have a pH of about 7.0. (Koxloff, Schofield and Lute, Ice Nucleating Activity of Pseudomonas syringae and Erwinia herbicola, J. Bacter. 153 pages 222-231 (1983)) In this reference, the microorganisms are not recovered in dry form and the suspensions are tested directly for activity. It is noted that the ice nucleating activity is not stable in the suspension and decreases overnight.

If the known procedures are used for the production of large volumes of the microorganisms, less than the desired ice nucleating activity (INA) is obtained. Not only is the ice nucleating activity of the initial suspension less than desired, but much of the acticity is lost during the freeze drying of large volumes of the material. The end result is a process that is not capable of producing commercial quantities of microorganism at reasonable cost.

In U.S. Patent Application Serial No. 910,600 filed September 23, 1986 (Your Ref.: A873045-EK), there is disclosed an improvement in the processes that were known in the art for the production of ice nucleating microorganisms. In this process, the pH is controlled so as to be between 6.5 and 5.5. As the pH approaches about 6.7, acid is added and as the pH approaches 5.5, base is added. Other improvements to the process for fermenting ice nucleating microorganisms are also disclosed in this application. For example, a preferred medium is disclosed which comprises mannitol as the carbon source and a yeast extract as the nitrogen source.

While the process described in the above application was a significant advance in the state of the art, still further improvements in the production of such microorganisms were sought. For example, mannitol is an expensive source of carbon. It would be desirable to use a cheaper source, such as glucose or sucrose. Still further improvements in the production of INA would be desirable.

The present invention is an improved method for the fermentation of a microorganism which has ice nucleating activity comprising the steps of fermenting the microorganism in a medium and recovering the microorganism. The improvement comprises using a medium comprising a sugar as the carbon source and $\alpha$-ketoglutarate or an $\alpha$-ketoglutarate yielding amino acid as the nitrogen source.

The medium used in the fermentation according to the present invention comprises two essential components, a sugar and $\alpha$-ketoglutarate or an $\alpha$-ketoglutarate yielding amino acid.

Sugars are useful in the present invention include glucose (or crude glucose such as dextrose), sucrose, fructose, erythrose, mannose, xylose and ribose. Commercial sources of these sugars can conveniently be used. Such sources include liquid sucrose, high fructose corn syrup and dextrose corn syrup. Mixtures of these sugars can also be used. Other carbon sources can be used in combination with these sugars such as mannitol and other sugar derivatives.

The other essential component is $\alpha$-ketoglutarate or an $\alpha$-ketoglutarate yielding amino acid. Amino acids which yield $\alpha$-ketoglutarate in biological processes are arginine, histidine, glutamine, glutamic acid and proline. A discussion of the production of $\alpha$-ketoglutarate from these amino acids is found in Biochemistry 2nd ed., Lehninger, Worth (1975) page 574 et seq. Mixtures of these compounds can also be used.

It is surprising that $\alpha$-ketoglutarate or an $\alpha$-ketoglutarate yielding amino acid could be used to advantage as the nitrogen source since other common nitrogen sources, such as simple ammonium salts, do not give similar results.

The medium also preferably contains phosphates such as potassium phosphates. A useful range of phosphate concentration is between 0.2 to 6 g/L preferably 0.6 to 3 g/L.

The media preferably contains other components. As is known in the art for the fermentation of these microorganisms, magnesium sulfate is preferred. Also, it is desirable for the medium to contain trace amounts of metals. We have found that trace amounts of iron and zinc are particularly useful. Other metals that are preferably added include manganese, sodium, calcium and molybdenum. By trace amounts, it is meant that the metal is present in a concentration of .0001 to 10 g/L. Concentration of the essential sugar and α-ketoglutarate or an α-ketoglutarate yielding amino acid is not critical. The currently preferred media contains 30 to 100 g/L sugar and 10 to 50 g/L α-ketoglutarate yielding amino acid.

The composition of the currently preferred media is as follows:

| | |
|---|---|
| glucose | 80 g/L |
| mannitol | 5 g/L |
| L—glutamic acid | 20 g/L |
| magnesium sulfate | 2 g/L |
| potassium phosphates | 2 g/L |
| Trace metals | |
| —iron sulfate | .056 g/L |
| —zinc sulfate | .0012 g/L |
| —Manganese sulfate (monohydrate) | .034 g/L |
| —sodium chloride | .012 g/L |
| —calcium chloride (dihydrate) | .002 g/L |
| —sodium molybdate (dihydrate) | .002 g/L |

During the fermentation, it is desirable to control the pH as disclosed in the above identified application. The temperature is preferably between 19°C and 25°C.

Any microorganism that has ice nucleation activity can be produced by the present invention. Suitable microorganisms include Pseudomonads such as P. syringae and P. fluorscens, P. coronafaciens and P. pisi. Other microorganisms that are useful in the present invention include Erwina herbicola. The presently preferred microorganism is P. syringae ATCC No. 53543 deposited on September 23, 1986 in accordance with the Budapest Treaty with the American Type Culture Collection in Rockville Maryland, USA.

The microorganism that is produced in the described fermentation can be dried in a number of ways. Spray drying and freeze drying are typical examples. Any drying process will reduce the INA to a certain extent. One preferred method that preserves a large amount of the INA that is produced in the fermentor is the process that is described in copending, commonly assigned United States Patent Application Serial No. 910,552, filed September 23, 1986 entitled "Recovery of Microorganisms Having Ice Nucleating Activity" of Lindsey. In this process, the medium is cooled, concentrated, run into a cryogenic liquid to form pellets and then the pellets are freeze dried at relatively low temperature.

In the examples presented below, the INA is calculated using conventional techniques. The INA is determined by placing a plurality of microorganism containing water droplets (10 μl) on paraffin coated aluminum foil. The foil is maintained at -5°C by placing it on a constant temperature bath. Details regarding this procedure are found in the literature, for example, Vali, Quantitative evaluation of Experimental Results on the Heterogenous Freezing of Sypercooled Liquids, J. Atoms Sci., 28, 402-409 (1971). The INA reported in the examples is the number of ice nucleating sites per dry gram of microorganism. For the present purposes, the INA is measured using a sample directly from the fermentor without drying. It will therefore be referred to as "Fermentor INA". The units are nuclei per dry gram of microorganism.

The following example is submitted for a further understanding of the invention.

Example

A 4 mL sample of <u>Pseudomonas syringae</u> ATCC No. 53543 was placed in a 300 L seed tank which contained 200 L of the medium defined in Table 1 of USSN 910,600. After 40 hours in the seed tank, the resulting broth was transferred to a 3,000 L fermentor containing media described above to a total volume of 2,000 L.

During all of these steps, the temperature was controlled at 24°C. The pH was controlled with 4N hydrochloric acid and 2N sodium hydroxide. The acid was added when the pH approached 6.6 and the base was added when the pH approached 5.6. The dissolved oxygen was maintained at greater than 30% saturation. Antifoaming agent was added as needed to control foaming.

After 22 hours in the 3,000 L fermentor, the cell mass reached 14.5 g/L. The fermentor INA was $1.0 \times 10^{12}$.

## Claims

1. In a method for the fermentation of a microorganism which has ice nucleating activity comprising the steps of fermenting the microorganism in a medium and recovering the microorganism, the improvement comprising using a medium comprising a sugar as the carbon source and $\alpha$-ketoglutarate or an $\alpha$-ketoglutarate yielding amino acid.

2. The method according to claim 1 wherein said sugar is glucose.

3. The method according to any previous claim wherein said $\alpha$-ketoglutarate yielding amino acid is L-glutamic acid.

4. The method according to any previous claim wherein said medium additionally contains trace amounts of iron and zinc.

5. The method according to any previous claim wherein said medium additionally contains phosphates in a concentration of between 0.2 to 6 g/L.

6. The method according to any previous claim wherein said microorganism is a <u>Pseudomonad</u>.

7. The method according to claim 6 wherein said microorganism is <u>P. syringae</u>.